# EUROPÄISCHE PATENTSCHRIFT

(11) **EP 0 495 033 B1**
(45) Veröffentlichungstag und Bekanntmachung des Hinweises auf die Patenterteilung: **31.08.1994**
(21) Anmeldenummer: 91913308.2
(22) Anmeldetag: 30.07.1991
(51) Int. Cl.: A61M 25/00

(54) **DIALYSESONDE**
DYALYSIS PROBE
SONDE DE DIALYSE

(30) Priorität: 30.07.1990 AT 1598/90
(43) Veröffentlichungstag der Anmeldung: 22.07.1992
(73) Patentinhaber: AVL Medical Instruments AG, 8207 Schaffhausen (CH)
(72) Erfinder: SKRABAL, Falko, A-8010 Graz (AT); KLEINHAPPL, Erich, A-8062 Weinitzen (AT)
(74) Vertreter: Krause, Walter, Dr. Dipl.-Ing.
(86) Internationale Anmeldenummer: AT9100092
(87) Internationale Veröffentlichungsnummer: WO9202270

(56) Entgegenhaltungen:
- EP-A- 41
- DE-A- 3 246 728
- FR-A- 2 398 485
- US-A- 4 180 068

## Beschreibung

Die Erfindung betrifft eine Dialysesonde mit einer zu- und abführenden Leitung für das Dialysemittel, welche eine Einführnadel zum Einführen der Sonde aufweist, die von einer biegsamen Kunststoffkanüle umgeben ist, welche ihrerseits zumindest zum Teil von einer schlauchförmigen Dialysemembran umgeben ist, wobei die Dialysemembran am distalen Ende mit der Kunststoffkanüle flüssigkeitsdicht verbunden ist.

Aus der DE-OS 33 42 170 ist eine Dialysesonde bekannt geworden, welche primär für das Einsetzen in biologisches Gewebe bestimmt ist und eine Dialysemembran sowie zu- und abführende Leitungen für das Dialysemittel umfaßt. Die Sonde weist ein Gehäuse auf, welches die Dialysemembran stützt und teilweise freigibt. Das Gehäuse besteht im wesentlichen aus einem Rahmen aus einer Metallhülse, in deren Wand eine Öffnung angeordnet ist, in deren Bereich die Membranoberfläche freiliegt und zur Dialyse genützt werden kann. Die Größe und die Form der Öffnung kann in Abhängigkeit von der gewünschten Größe und Form der Dialyseoberfläche der Membran unterschiedlich sein, wird jedoch immer von Teilen der Metallhülse beschränkt. Die Dialysemembran wird so in den Rahmen eingesetzt, daß sie dicht an der Wand des Rahmens anliegt, wobei die distalen Enden der Dialysemembran und des Rahmens beispielsweise durch Epoxyharz miteinander verklebt werden. Das distale Ende der Sonde ist mit einem den Innenraum der Sonde begrenzenden halbrunden Stopfen verschlossen. Als zu- und abführende Leitungen werden dünnwandige Metallrohre verwendet, welche durch eine Dichtung am proximalen Ende der Sonde ragen. Die Öffnung der einen Leitung sitzt in der Nähe des distalen Endes, während die Öffnung der anderen Leitung innerhalb der Sonde in der Nähe des proximalen Endes des Membranabschnittes liegt.

Durch den als Metallhülse ausgeführten Rahmen, weist die Sonde die für das Einführen nötige mechanische Festigkeit auf, was jedoch bei Verweilsonden für manche Anwendungen den Tragekomfort entscheidend mindert. Ein weiterer Nachteil besteht darin, daß nur relativ geringe Teile der Sondenoberfläche für die eigentliche Dialyse zur Verfügung stehen.

Eine Dialysesonde der eingangs genannten Art ist beispielsweise aus der US-PS 4 774 955 bzw. deren Fig. 4 und 5 bekannt geworden. Diese Sonde weist eine nach dem Einführen der Sonde entfernbare Hohlnadel auf, welche von einer Kunststoffkanüle umgeben ist. Die Kunststoffkanüle wird ihrerseits von einer schlauchförmigen Dialysemembran umgeben, welche distal und proximal flüssigkeitsdicht mit der Kunststoffkanüle verbunden ist. Durch eine einzige Zuleitung kann in das Lumen zwischen Kunststoffkanüle und Dialysemembran eine Flüssigkeit eingebracht werden, welche zur Analyse wieder durch diese Zuleitung abgepumpt wird. Eine kontinuierliche Dialyse ist somit mit dieser Sonde nicht möglich.

Aus der FR-A 2 398 485 ist weiters eine Dialysesonde bekannt, bei welcher ein zuführender Kanal in der Sonde bis knapp an deren Spitze geführt wird und dort in einen, außenseitig von der Dialysemembran gebildeten Ringraum eintritt, sodaß das Dialysemittel den Ringraum aufsteigend füllt und schließlich über eine separate Abführleitung die Sonde in Richtung Analysator wieder verläßt. Dieser Aufbau dient vor allem dem Zweck der kontinuierlichen Erzeugung von Probenmaterial.

Schließlich ist aus der EP-A 0 000 041 ein intravaskulärer Katheter ("single-needle-Typ") bekannt, der in seinem distalen und mittleren Abschnitt innenliegend zwei getrennte, aber konzentrisch angeordnete Lumina aufweist, welche im proximalen Teil des Katheters in einen Griffteil einmünden und über zwei getrennte Kanäle in die beiden getrennten Katheteranschlüsse übergehen. Die Katheteranschlüsse dienen als Verbindung zu einer Dialyseeinrichtung.

Aufgabe der vorliegenden Erfindung ist es, ausgehend von bekannten, eingangs beschriebenen Sonden, eine Dialysesonde vorzuschlagen, welche einfach zu applizieren ist, einen guten Trage- bzw. Liegekomfort aufweist und bei welcher ein ständiger Kreislauf des Dialysemittels gewährleistet ist.

Diese Aufgabe wird erfindungsgemäß dadurch gelöst, daß ein Griffteil vorgesehen ist, welcher separate zu- und abführende Leitungen für das Dialysemittel aufweist, sowie daß in der Sonde zumindest zwei mit den zu- und abführenden Leitungen verbundene Lumen geführt sind, wovon zumindest ein Lumen von der Dialysemembran begrenzt ist, daß das nach dem Entfernen der Einführnadel freiwerdende Lumen der Kunststoffkanüle mit einer der zu- und abführenden Leitungen verbunden ist, und das Lumen zwischen Kunststoffkanüle und Dialysemembran mit der anderen zu- und abführenden Leitung in Verbindung steht, sowie daß das distale Ende der Kunststoffkanüle eine seitliche Wandöffnung zum Lumen zwischen Kunststoffkanüle und Dialysemembran aufweist. Durch die distale Verbindungsöffnung wird im Zusammenhang mit den separaten zu- und abführenden Leitungen für einen stetigen Fluß des Dialysemittels gesorgt, wobei die Sonde einfach und relativ schmerzfrei zu applizieren ist. Weiters wird damit eine Dialysesonde realisiert, deren im Körper verbleibende Teile aus weichen, biegsamen Materialien hergestellt sind, wobei die gesamte Oberfläche der Dialysesonde für die eigentliche Dialyse genutzt wird.

Eine weitere Ausführungsvariante der Erfindung ist dadurch gegeben, daß ein Griffteil vorgesehen ist, welcher separate zu- und abführende Leitungen für das Dialysemittel aufweist, sowie daß in der Sonde zumindest zwei mit den zu- und abführenden Leitungen verbundene Lumen geführt sind, wovon zumindest ein Lumen von der Dialysemembran begrenzt ist, daß das Lumen zwischen Kunststoffkanüle und Dialysemembran im wesentlichen in Längerichtung der Dialysesonde geteilt ausgeführt ist, sodaß zwei Lumen mit im wesentlichen halbmondförmigem Querschnitte entstehen, welche beiden Lumen im Bereich des distalen Endes der Kunststoffkanüle eine Strömungsverbindung aufweisen und mit je einer der separaten zu- und abführenden Leitungen in Verbindung stehen, sowie daß das nach dem Entfernen der Einführnadel freiwerdende Lumen der Kunststoffkanüle mit einer Medikamentenzuleitung im Griffteil verbindbar ist. Ein zusätzlicher Vorteil dieser Ausführungsvariante besteht darin, daß das innere Lumen der Kunststoffkanüle nach dem Herausziehen der Einführnadel für die vom übrigen Dialysesystem unabhängige Zufuhr eines Medikaments verwendet werden kann, wie dies beispielsweise bei der eingangs genannten DE-OS 33 42 170 nicht möglich ist.

Erfindungsgemäß läßt sich die zuletzt genannte Ausführungsvariante z.B. dadurch realisieren, daß die Dialysemembran durch zwei im wesentlichen in Längsrichtung der Dialysesonde verlaufende Klebe- oder Schweißstellen und eine distale, ringförmige Klebe- oder Schweißstelle mit der Kunststoffkanüle verbunden ist, wobei zwischen den in Längsrichtung verlaufenden Klebe- oder Schweißstellen und der ringförmigen Klebe- oder Schweißstelle zumindest eine Lücke besteht, durch welche die Strömungsverbindung zwischen den beiden Lumen hergestellt ist, bzw. daß die Dialysemembran an zwei im wesentlichen in Längsrichtung der Dialysesonde verlaufende Stege und ggf. an einem distalen ringförmigen Steg der Kunststoffkanüle befestigt ist, wobei zwischen den in Längsrichtung verlaufenden Stegen und dem ringförmigen Steg zumindest eine Lücke besteht, durch welche die Strömungsverbindung zwischen den beiden Lumen hergestellt ist. Der distale Steg soll vorzugsweise so geformt sein, daß er an den harten Kern der Sonde stufenlos anschließt.

Für die Kunststoffkanüle können Polyolefine (Polypropylen, Polyäthylen) verwendet werden, welche nach einem Oxidationsvorgang mittels Epoxyharzen oder Polycyanaten mit der Dialysemembran verklebt werden.

Falls sich die Materialien der Kunststoffkanüle und der Dialysemembran für Klebe- oder Schweißprozesse nicht eignen, kann erfindungsgemäß vorgesehen sein, daß die Dialysemembran an den in Längsrichtung verlaufenden Stegen und ggf. am ringförmigen Steg mechanisch, vorzugsweise durch Klemmen, befestigt ist. Die Klemmwirkung kann beispielsweise durch eine Nut oder durch eine Verzahnung der Stege erreicht werden.

In einer weiteren vorteilhaften Ausführungsvariante der Erfindung ist vorgesehen, daß ein Griffteil vorgesehen ist, welcher separate zu- und abführende Leitungen für das Dialysemittel aufweist, sowie daß in der Sonde zumindest zwei mit den zu- und abführenden Leitungen verbundene Lumen geführt sind, wovon zumindest ein Lumen von der Dialysemembran begrenzt ist, daß die Kunststoffkanüle ein nach dem Entfernen der Einführnadel freiwerdendes inneres Lumen aufweist, welches mit einer Medikamentenzuleitung verbunden ist, daß in der Wand der Kunststoffkanüle ein weiteres Lumen vorgesehen ist, welches mit einer der zu- und abführenden Leitungen in Verbindung steht, sowie daß das Lumen (12') zwischen Kunststoffkanüle und Dialysemembran mit der anderen zu- und abführenden Leitung verbunden ist, wobei das Lumen in der Wand der Kunststoffkanüle zumindest eine Wandöffnung zu dem von der Dialysemembran begrenzten Lumen aufweist. Auch bei dieser Ausführungsvariante handelt es sich um ein geschlossenes Dialysesystem, welches sich zusätzlich zur Medikamentenzufuhr eignet.

Bei Anwendungen, wo ein Verbleiben der Einführnadel in der Sonde in Kauf genommen werden kann, ist vorgesehen, daß ein Griffteil vorgesehen ist, welcher separate zu- und abführende Leitungen für das Dialysemittel aufweist, sowie daß in der Sonde zumindest zwei mit den zu- und abführenden Leitungen verbundene Lumen geführt sind, wovon zumindest ein Lumen von der Dialysemembran begrenzt ist, daß das Lumen zwischen Kunststoffkanüle und Dialysemembran mit einer der zu- und abführenden Leitungen in Verbindung steht, daß die Einführnadel bis zu einer distalen seitlichen Wandöffnung als Hohlnadel ausgeführt ist, welche am Griffende die andere der zu- und abführenden Leitungen aufweist, wobei die Einführnadel nach dem Einführen der Sonde zurückziehbar ist, bis deren seitliche Wandöffnung mit einer distalen Wandöffnung der Kunststoffkanüle zur Deckung kommt.

Eine derartige Sonde kann zusätzlich zum Applizieren eines Medikamentes verwendet werden, wenn die Kunststoffkanüle in einem distalen, membranfreien Bereich eine weitere seitliche Wandöffnung aufweist, welche nach einer axialen Verschiebung der Einführnadel mit deren Wandöffnung zur Deckung kommt.

Erfindungsgemäß sind dabei Mittel für die axiale Positionierung der Einführnadel, sowie Elemente zur Sicherung gegen das Verdrehen der Einführnadel vorgesehen.

Eine Weiterbildung der Erfindung sieht vor, daß der Griffteil der Dialysesonde die Kunststoffkanüle in einer zentralen Bohrung aufnimmt, wobei der Griffteil sich in Richtung distales Ende der Kunststoffkanüle verjüngt, sodaß die Dialysemembran über die Außenfläche des Griffteiles schiebbar und dort befestigbar ist.

Um das Aufschieben der Dialysemembran auf das Griffstück zu erleichtern, kann die Dialysemembran erfindungsgemäß am dem Griffteil zugewandten Ende einen größeren Durchmesser aufweisen als am distalen Ende der Kunststoffkanüle.

Herstellungstechnisch ist es besonders vorteilhaft, wenn die zentrale Bohrung des Griffteiles Aussparungen aufweist, welche gemeinsam mit der eingesetzten Kunststoffkanüle Stromungsverbindungen zwischen mindestens einer der zu- und abführenden Leitungen und zumindest einem Lumen zwischen Kunststoffkanüle und Dialysemembran bilden. Die inneren Strömungsverbindungen des Griffteiles können dabei bereits bei der Herstellung beispielsweise als Spritzgußteil berücksichtigt werden.

Weiters kann es von Vorteil sein, wenn die Dialysemembran in dem Bereich, wo sie am Griffstück befestigt ist, nicht straff gespannt ist, sondern Falten aufweist, um ein Einreißen der Dialysemembran bei Verbiegung der Kunststoffkanüle zu verhindern.

Die Dialysesonde wird bis zu dem mit der Dialysemembran bedeckten Bereich des Griffteiles eingeführt, sodaß Luftzutritt bzw. Austritt des Dialysemittels verhindert wird.

Obwohl die Dialysesonde nach dem Herausziehen der Einführnadel händisch, beispielsweise durch einen Stopfen, verschlossen werden kann, ist in einer weiteren Ausgestaltung der Erfindung vorgesehen, daß der Griffteil der Dialysesonde einen von der Einführnadel durchsetzten elastischen Stopfen aufnimmt, der die Dialysesonde nach dem Entfernen der Einführnadel im Bereich des Griffteiles selbsttätig verschließt.

Schließlich kann erfindungsgemäß die Dialysemembran aus dehnbarem Material bestehen und durch den Druck des Dialysemittels elastisch verformbar sein. Dadurch kann die aktive Fläche der Membran noch nach dem Einführen der Sonde vergrößert werden. Durch Variation des Druckes des Dialysemittels kann auch die Porengröße der Dialysemembran variiert werden.

Beispielsweise kann eine Cellophanmembran dicht auf die Kunststoffkanüle aufgezogen werden, wobei erst bei Kontakt mit dem Dialysemittel eine Quellung der Membran bis zu 20 % erfolgt und das Lumen für den Dialysestrom gebildet wird.

Da die Kunststoffkanüle und die Dialysemembran aus ähnlichen oder kompatiblen Materialen bestehen können (erfindungsgemäß aus Cellophan, Cuprophan, Polyurethan, Polykarbonat bzw. Poyurethan mit eingebautem Polyäther od. ähnliches), können diese sehr leicht miteinander verschweißt oder verklebt werden.

Im folgenden wird die Erfindung anhand von Zeichnungen näher erläutert. Es zeigen:
- Fig. 1: eine Dialysesonde nach der Erfindung in schematischer Darstellung,
- Fig. 2: einen Schnitt im distalen Bereich der Sonde entlang der Linie II-II in Fig. 1, die
- Fig. 3, 5 und 7: Ausführungsvarianten der erfindungsgemäßen Sonde, die
- Fig. 4, 6 und 8: Schnitte der Ausführungsvarianten in einer Fig. 2 entsprechenden Schnittebene,
- Fig. 9: eine weitere Ausführungsvariante in einer Fig. 2 entsprechenden Schnittebene,
- Fig. 10: eine Ausführungsvariante nach Fig. 3,
- Fig. 11: einen Schnitt entlang der Linie XI-XI in Fig. 10 und
- Fig. 12: eine Ausführungsvariante nach Fig. 1.

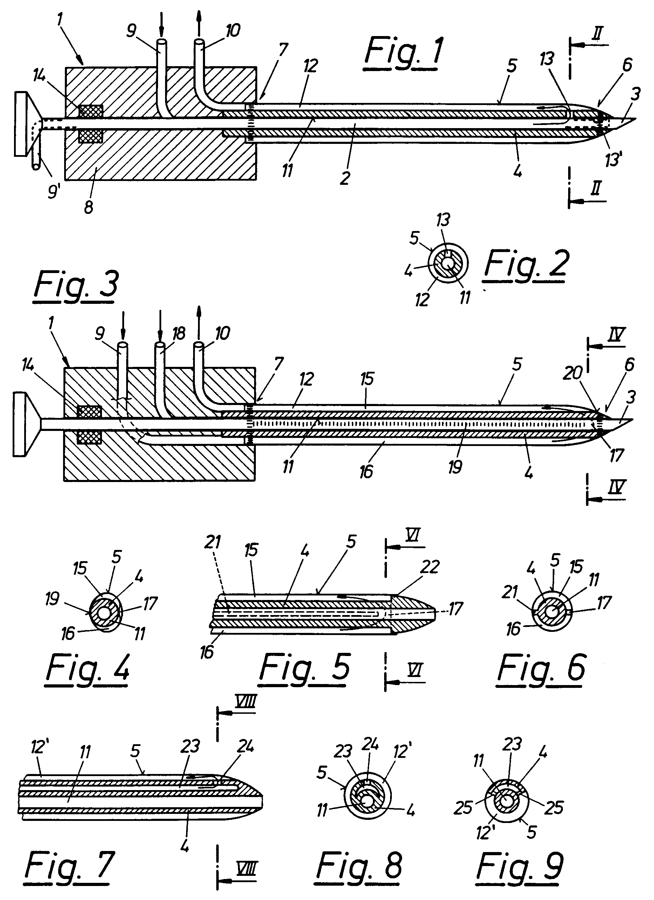

Die in Fig. 1 und 2 dargestellte Ausführungsvariante einer Dialysesonde 1, weist eine Einführnadel 2 auf, welche von einer das geschliffene bzw. spitze Ende 3 freilassenden, biegsamen Kunststoffkanüle 4 umgeben ist. Die Kunststoffkanüle 4 ist von einer schlauchförmigen Dialysemembran 5 umgeben, welche am distalen Ende 6 mit der Kunststoffkanüle und am anderen Ende 7 mit einem Griffteil 8 der Dialysesonde 1 flüssigkeitsdicht verbunden ist. Die Verbindung kann als Klebestelle oder Schweißstelle ausgeführt sein. Der Griffteil 8 weist zu- und abführende Leitungen 9, 10 für das Dialysemittel auf, wobei die zuführende Leitung 9 beispielsweise in das nach dem Entfernen der Einführnadel 2 freiwerdende Lumen 11 der Kunststoffkanüle 4 mündet und die abführende Leitung 10 mit dem Lumen 12 zwischen Kunststoffkanüle 4 und schlauchförmiger Dialysemembran 5 in Verbindung steht. Am distalen Ende 6 der Kunststoffkanüle 4 befindet sich eine seitliche Wandöffnung 13, welche zum äußeren Lumen 12 führt und eine Strömungsverbindung für das Dialysemittel herstellt. Der Austritt von Dialyseflüssigkeit am distalen Ende der Sonde wird hier durch das anliegende Gewebe verhindert. Der Griffteil 8 der Dialysesonde weist einen von der Einführnadel 2 durchsetzten, elastischen Stopfen 14 auf, welcher die Dialysesonde 1 bzw. das innere Lumen 11 der Kunststoffkanüle 4 nach dem Entfernen der Einführnadel 2 selbsttätig verschließt.

Im Zusammenhang mit Fig. 1 ist auch eine Ausführungsvariante denkbar, bei welcher die Einführnadel 2 bis zu einer distalen seitlichen Wandöffnung 13' als Hohlnadel ausgeführt ist, welche am Griffende eine zu- oder abführende Leitung 9' aufweist. Nach dem Einführen der Sonde wird die Einführnadel 2 zurückgezogen, bis die Wandöffnungen 13 und 13' zur Deckung kommen und so ein geschlossener Kreislauf für das Dialysemittel geschaffen wird. Die Leitung 9 im Griffstück 8 kann dann natürlich entfallen.

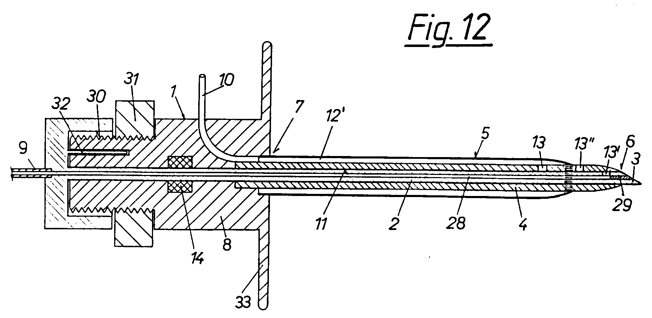

Zur genauen axialen Positionierung der Einführnadel kann diese eine nicht weiter dargestellte ringförmige Nut im Griffbereich aufweisen, in welche in oder am Griffteil angeordnete Noppen oder dgl. eingreifen. Die Einführnadel 2 kann auch vorgeschoben werden bis die Wandöffnung 13' von der Kunststoffkanüle 4 freigegeben wird. In dieser Stellung kann ein Medikament über die Leitung 9' zugeführt werden. Eine Ausführungsvairante, bei welcher die Einführnadel 2 in der Sonde verbleibt, wird in der später noch ausführlicher beschriebenen Fig. 12 dargestellt.

Die Ausführungsvariante nach Fig. 3 und 4 zeigt eine Dialysesonde mit völlig geschlossenem Kreislauf für das Dialysemittel. Das Lumen 12 zwischen Kunststoffkanüle 4 und Dialysemembran 5 ist hier in Längsrichtung der Dialysesonde durch zwei Klebe- oder Schweißstellen geteilt ausgeführt, sodaß zwei Lumen 15, 16 mit aus Fig. 4 bzw. 6 ersichtlichem, im wesentlichen halbmondförmigen Querschnitten entstehen. Die beiden Lumen 15, 16 weisen im Bereich des distalen Endes 6 eine Strömungsverbindung 17 auf, wodurch - wie durch einen Pfeil angedeutet - die Strömungsrichtung des Dialysemittels innerhalb der Sonde umgekehrt wird. Das Lumen 16 ist beispielsweise mit der zuführenden Leitung 9 und das Lumen 15 mit der abführenden Leitung 10 verbunden. Das innere Lumen 11 der Kunststoffkanüle 4 wird hier für die Führung des Dialysemittels nicht benötigt und ist mit einer ebenfalls im Griffteil 8 angeordneten Medikamentenzuleitung 18 verbunden.

Wie in den Fig. 3 und 4 ausgeführt, kann die Dialysemembran 5 durch zwei in Längsrichtung der Dialysesonde 1 verlaufende Klebe- oder Schweißstellen 19 und eine distale, ringförmige Klebe- oder Schweißstelle 20 (bzw. mechanische Klemmstellen) mit der Kunststoffkanüle 4 verbunden sein. Zwischen zumindest einer der in Längsrichtung verlaufenden Klebe- oder Schweißstellen 19 und der ringförmigen Klebe- oder Schweißstelle 20 befindet sich eine Lücke, durch welche die Strömungsverbindung 17 zwischen den beiden Lumen 15 und 16 realisiert wird.

Die Ausführungsvariante nach den Fig. 5 und 6 unterscheidet sich von jener nach Fig. 3 und 4 nur dadurch, daß die Zweiteilung des äußeren Lumens in die beiden Lumen 15 und 16 durch im wesentlichen in Längsrichtung verlaufende Stege 21 realisiert wird. Es kann auch ein radialer Steg 22 vorhanden sein, wobei zumindest einer der Stege 21 nicht mit dem radialen Steg verbunden ist und eine Lücke für die Strömungsverbindung 17 zwischen den beiden Lumen 15 und 16 freiläßt. Die Längsteilung durch Stege 21 oder Klebe- bzw. Schweißstellen 19 kann auch spiralig ausgeführt sein, um die Dialysestrecke zu verlängern.

Die Ausführungsvariante nach Fig. 7 und 8 zeigt ebenfalls ein geschlossenes Dialysesystem, bei welchem die Kunststoffkanüle 4 neben dem Lumen 11 noch ein weiteres Lumen 23 in der Wand der Kunststoffkanüle 4 aufweist. Das Lumen 23 ist beispielsweise mit der hier nicht dargestellten zuführenden Leitung für das Dialysemittel verbunden, wobei eine Wandöffnung 24 zu dem von der schlauchförmigen Dialysemembran 5 begrenzten äußeren Lumen 12' vorgesehen ist.

Schließlich kann entsprechend einer Ausführungsvariante nach Fig. 9 die Dialysemembran 5 die Kunststoffkanüle 4 nur zum Teil umgeben. Die Zufuhr des Dialysemittels erfolgt hier z.B. durch das Lumen 23 in der Wand der Kanüle 4 und die Abfuhr durch das äußere Lumen 12'. Die Strömungsverbindung im distalen Bereich wird hier durch Öffnungen 25 relisiert. Das Lumen 11 steht auch hier für die Medikamentenzuleitung zur Verfügung.

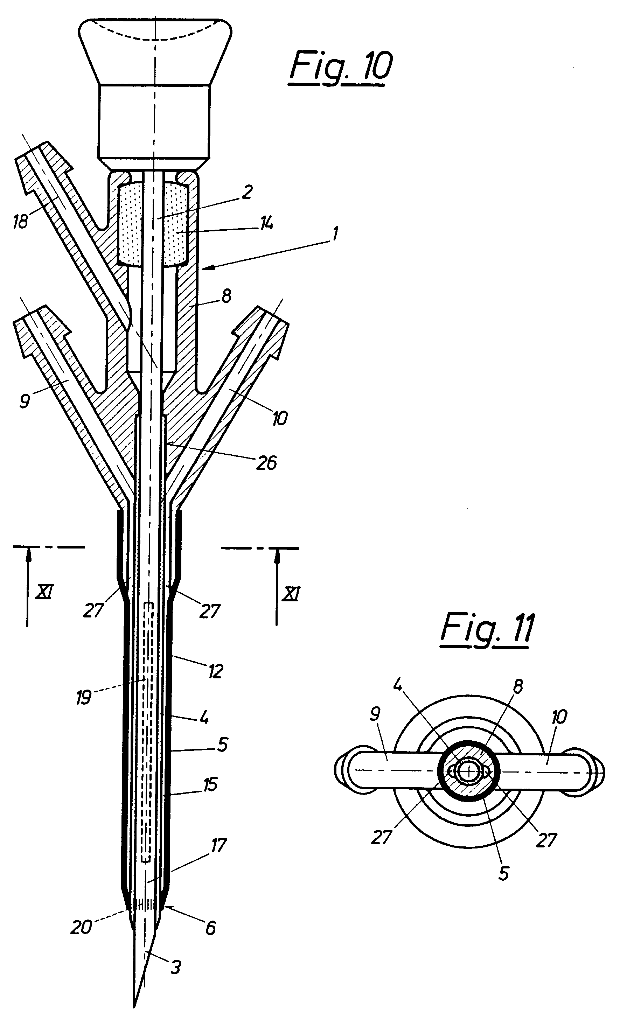

Die in Fig. 10 und 11 dargestellte Ausführungsvariante eignet sich besonders gut für die Serienherstellung. Der Griffteil 8 der Sonde ist z.B. ein Spritzgußteil, welcher die Kunststoffkanüle 4 in einer zentralen Bohrung 26 aufnimmt. Der Griffteil 8 verjüngt sich in Richtung distales Ende 6 der Kunststoffkanüle 4, wodurch die Dialysemembran 5 über die Außenfläche des Griffteiles geschoben und dort durch Kleben, Schweißen oder Klemmen befestigt werden kann. Um das Aufschieben auf das Griffstück 8 zu erleichtern, weist die Dialysemembran 5 in diesem Bereich einen erweiterten Durchmesser auf. Die zentrale Bohrung 26 weist seitliche Aussparungen 27 auf, welche gemeinsam mit der in die Bohrung 26 eingesetzten Kunststoffkanüle 4 Strömungsverbindungen bilden, welche einerseits die zuführende Leitung 9 mit dem Lumen 16 und anderseits das Lumen 15 mit der abführenden Leitung 10 verbinden. Die Dialysesonde nach Fig. 10 wird bis zu dem mit der Dialysemembran 5 bedeckten Bereich des Griffteiles 8 eingeführt.

Die Ausführungsvariante nach Fig. 12 zeigt eine Dialysesonde 1, bei welcher die Einführnadel 2 bis zu einer distalen, seitlichen Wandöffnung 13' als Hohlnadel ausgeführt ist. Am Griffende der Hohlnadel ist die zuführende Leitung 9 angeordnet, am anderen Ende weist das Lumen 28 der Nadel einen Verschluß 29 auf. Auch hier wird die Einführnadel 2 nach dem Einführen der Sonde zurückgezogen, bis deren seitliche Wandöffnung 13' mit der distalen Wandöffnung 13 der Kunststoffkanüle 4 zur Deckung kommt, um den Dialysekreislauf herzustellen. Die Kunststoffkanüle 4 weist in einem distalen, membranfreien Bereich eine weitere, seitliche Wandöffnung 13'' auf, welche nach einer axialen Verschiebung der Einführnadel 2 mit deren Wandöffnung 13' zur Deckung gebracht werden kann. In dieser Stellung ist die Zuführung eines Medikamentes möglich.

Als Mittel für die axiale Positionierung der Einführnadel 2 kann beispielsweise ein Gewinde 30 am Griffteil 8 im Zusammenhang mit einer auf das Griffstück der Nadel wirkenden Einstellmutter 31 dienen. Die Einstellmutter 31 und der Griffteil 8 können Markierungen aufweisen, welche die axiale Stellung der Einführnadel 2 anzeigen. Es sind jedoch auch alle anderen bekannten Positionierungsmittel denkbar.

Zur Sicherung gegen das Verdrehen der Einführnadel 2 in der Kunststoffkanüle 4 ist ein Sicherungselement 32 vorgesehen. Die Auflagefläche 33 sorgt dafür, daß die Sonde gut am Körper fixiert werden kann.

Schließlich ist es bei dieser Ausführungsvariante auch denkbar, die Einführnadel 2 nach dem Einführen der Sonde zu entfernen und durch einen relativ weichen Mandrin mit einem Lumen 28 zu ersetzen, welcher die Funktionen der Hohlnadel übernimmt.

Die wesentlichen Teile der Kunststoffkanüle 4 der einzelnen Ausführungsvarianten - inkl. Lumen 23 und Längsstege 21 - lassen sich maschinell z.B. durch Extrusion herstellen und müssen nur minimal durch Bohren der Öffnungen 13 bzw. 24, 25, durch Überziehen mit der Dialysemembran und Befestigen des Griffteiles 8 bearbeitet werden.

## Patentansprüche

1. Dialysesonde (1) mit einer zu- und abführenden Leitung (9, 10) für das Dialysemittel, welche eine Einführnadel (2) zum Einführen der Sonde aufweist, die von einer biegsamen Kunststoffkanüle (4) umgeben ist, welche ihrerseits zumindest zum Teil von einer schlauchförmigen Dialysemembran (5) umgeben ist, wobei die Dialysemembran (5) am distalen Ende mit der Kunststoffkanüle (4) flüssigkeitsdicht verbunden ist, **dadurch gekennzeichnet**, daß ein Griffteil (8) vorgesehen ist, welcher separate zu- und abführende Leitungen (9, 10) für das Dialysemittel aufweist, sowie daß in der Sonde zumindest zwei mit den zu- und abführenden Leitungen (9, 10) verbundene Lumen (11, 12) geführt sind, wovon zumindest ein Lumen von der Dialysemembran (5) begrenzt ist, daß das nach dem Entfernen der Einführnadel (2) freiwerdende Lumen (11) der Kunststoffkanüle (4) mit einer der zu- und abführenden Leitungen (9) verbunden ist, und das Lumen (12) zwischen Kunststoffkanüle (4) und Dialysemembran (5) mit der anderen zu- und abführenden Leitung (10) in Verbindung steht, sowie daß das distale Ende (6) der Kunststoffkanüle (4) eine seitliche Wandöffnung (13) zum Lumen (12) zwischen Kunststoffkanüle (4) und Dialysemembran (5) aufweist.

2. Dialysesonde (1) mit einer zu- und abführenden Leitung (9, 10) für das Dialysemittel, welche eine Einführnadel (2) zum Einführen der Sonde aufweist, die von einer biegsamen Kunststoffkanüle (4) umgeben ist, welche ihrerseits zumindest zum Teil von einer schlauchförmigen Dialysemembran (5) umgeben ist, wobei die Dialysemembran (5) am distalen Ende mit der Kunststoffkanüle (4) flüssigkeitsdicht verbunden ist, **dadurch gekennzeichnet**, daß ein Griffteil (8) vorgesehen ist, welcher separate zu- und abführende Leitungen (9, 10) für das Dialysemittel aufweist, sowie daß in der Sonde zumindest zwei mit den zu- und abführenden Leitungen (9, 10) verbundene Lumen (15, 16) geführt sind, wovon zumindest ein Lumen von der Dialysemembran (5) begrenzt ist, daß das Lumen zwischen Kunststoffkanüle (4) und Dialysemembran (5) im wesentlichen in Längerichtung der Dialysesonde (1) geteilt ausgeführt ist, sodaß zwei Lumen (15, 16) mit im wesentlichen halbmondförmigem Querschnitte entstehen, welche beiden Lumen (15, 16) im Bereich des distalen Endes (6) der Kunststoffkanüle (4) eine Strömungsverbindung (17) aufweisen und mit je einer der separaten zu- und abführenden Leitungen (9, 10) in Verbindung stehen, sowie daß das nach dem Entfernen der Einführnadel (2) freiwerdende Lumen (11) der Kunststoffkanüle (4) mit einer Medikamentenzuleitung (18) im Griffteil (8) verbindbar ist.

3. Dialysesonde nach Anspruch 2, **dadurch gekennzeichnet**, daß die Dialysemembran (5) durch zwei im wesentlichen in Längsrichtung der Dialysesonde (1) verlaufende Klebe- oder Schweißstellen (19) und eine distale, ringförmige Klebe- oder Schweißstelle (20) mit der Kunststoffkanüle (4) verbunden ist, wobei zwischen den in Längsrichtung verlaufenden Klebe- oder Schweißstellen (19) und der ringförmigen Klebe- oder Schweißstelle (20) zumindest eine Lücke besteht, durch welche die Strömungsverbindung (17) zwischen den beiden Lumen (15, 16) hergestellt ist.

4. Dialysesonde nach Anspruch 2, **dadurch gekennzeichnet**, daß die Dialysemembran (5) an zwei im wesentlichen in Längsrichtung der Dialysesonde (1) verlaufende Stege (21) und an ggf. einem distalen ringförmigen Steg (22) der Kunststoffkanüle (4) befestigt ist, wobei zwischen den in Längsrichtung verlaufenden Stegen (21) und dem ringförmigen Steg (22) zumindest eine Lücke besteht, durch welche die Strömungsverbindung (17) zwischen den beiden Lumen (15, 16) hergestellt ist.

5. Dialysesonde nach Anspruch 4, **dadurch gekennzeichnet**, daß die Dialysemembran (5) an den in Längsrichtung verlaufenden Stegen (21) und ggf. am ringförmigen Steg (22) mechanisch, vorzugsweise durch Klemmen, befestigt ist.

6. Dialysesonde (1) mit einer zu- und abführenden Leitung (9,10) für das Dialysemittel, welche eine Einführnadel (2) zum Einführen der Sonde aufweist, die von einer biegsamen Kunststoffkanüle (4) umgeben ist, welche ihrerseits zumindest zum Teil von einer schlauchförmigen Dialysemembran (5) umgeben ist, wobei die Dialysemembran (5) am distalen Ende mit der Kunststoffkanüle (4) flüssigkeitsdicht verbunden ist, **dadurch gekennzeichnet**, daß ein Griffteil (8) vorgesehen ist, welcher separate zu- und abführende Leitungen (9, 10) für das Dialysemittel aufweist, sowie daß in der Sonde zumindest zwei mit den zu- und abführenden Leitungen (9, 10) verbundene Lumen (23, 12') geführt sind, wovon zumindest ein Lumen von der Dialysemembran (5) begrenzt ist, daß die Kunststoffkanüle (4) ein nach dem Entfernen der Einführnadel (2) freiwerdendes inneres Lumen (11) aufweist, welches mit einer Medikamentenzuleitung (18) verbunden ist, daß in der Wand der Kunststoffkanüle (4) ein weiteres Lumen (23) vorgesehen ist, welches mit einer der zu- und abführenden Leitungen (9) in Verbindung steht, sowie daß das Lumen (12') zwischen Kunststoffkanüle (4) und Dialysemembran (5) mit der anderen zu- und abführenden Leitung (10) verbunden ist, wobei das Lumen (23) in der Wand der Kunststoffkanüle (4) zumindest eine Wandöffnung (24) zu dem von der Dialysemembran (5) begrenzten Lumen (12') aufweist.

7. Dialysesonde (1) mit einer zu- und abführenden Leitung (9,10) für das Dialysemittel, welche eine Einführnadel (2) zum Einführen der Sonde aufweist, die von einer biegsamen Kunststoffkanüle (4) umgeben ist, welche ihrerseits zumindest zum Teil von einer schlauchförmigen Dialysemembran (5) umgeben ist, wobei die Dialysemembran (5) am distalen Ende mit der Kunststoffkanüle (4) flüssigkeitsdicht verbunden ist, **dadurch gekennzeichnet**, daß ein Griffteil (8) vorgesehen ist, welcher separate zu- und abführende Leitungen (9, 10) für das Dialysemittel aufweist, sowie daß in der Sonde zumindest zwei mit den zu- und abführenden Leitungen (9, 10) verbundene Lumen (28, 12') geführt sind, wovon zumindest ein Lumen von der Dialysemembran (5) begrenzt ist, daß das Lumen (12') zwischen Kunststoffkanüle (4) und Dialysemembran (5) mit einer der zu- und abführenden Leitungen (10) in Verbindung steht, daß die Einführnadel (2) bis zu einer distalen seitlichen Wandöffnung (13') als Hohlnadel ausgeführt ist, welche am Griffende die andere der zu- und abführenden Leitungen (9) aufweist, wobei die Einführnadel (2) nach dem Einführen der Sonde zurückziehbar ist, bis deren seitliche Wandöffnung (13') mit einer distalen Wandöffnung (13) der Kunststoffkanüle (4) zur Deckung kommt.

8. Dialysesonde nach Anspruch 7, **dadurch gekennzeichnet**, daß die Kunststoffkanüle (4) in einem distalen, membranfreien Bereich eine weitere seitliche Wandöffnung (13'') aufweist, welche nach einer axialen Verschiebung der Einführnadel (2) mit deren Wandöffnung (13') zur Deckung kommt.

9. Dialysesonde nach Anspruch 7 oder 8, **dadurch gekennzeichnet**, daß Mittel (30, 31) für die axiale Positionierung der Einführnadel (2) sowie Elemente (32) zur Sicherung gegen das Verdrehen der Einführnadel vorgesehen sind.

10. Dialysesonde nach einem der Ansprüche 1 bis 9, **dadurch gekennzeichnet**, daß der Griffteil (8) der Dialysesonde (1) die Kunststoffkanüle (4) in einer zentralen Bohrung (26) aufnimmt, wobei der Griffteil (8) sich in Richtung distales Ende (6) der Kunststoffkanüle (4) verjüngt, sodaß die Dialysemembran (5) über die Außenfläche des Griffteiles (6) schiebbar und dort befestigbar ist.

11. Dialysesonde nach Anspruch 10, **dadurch gekennzeichnet**, daß die zentrale Bohrung (26) des Griffteiles (8) Aussparungen (27) aufweist, welche gemeinsam mit der eingesetzten Kunststoffkanüle (4) Strömungsverbindungen zwischen mindestens einer der zu- und abführenden Leitungen (9, 10) und zumindest einem Lumen (12; 12'; 15, 16) zwischen Kunststoffkanüle (4) und Dialysemembran (5) bilden.

12. Dialysesonde nach Anspruch 10 oder 11, **dadurch gekennzeichnet**, daß die Dialysemembran (5) am, dem Griffteil (8) zugewandten Ende einen größeren Durchmesser aufweist als am distalen Ende (6) der Kunststoffkanüle (4).

13. Dialysesonde nach einem der Ansprüche 1 bis 12, **dadurch gekennzeichnet**, daß der Griffteil (8) der Dialysesonde (1) einen von der Einführnadel (2) durchsetzten elastischen Stopfen (14) aufnimmt, der die Dialysesonde (1) nach dem Entfernen der Einführnadel (2) im Bereich des Griffteiles (8) selbsttätig verschließt.

14. Dialysesonde nach einem der Ansprüche 1 bis 13, **dadurch gekennzeichnet**, daß die Dialysemembran (5) aus dehnbarem Material besteht und durch den Druck des Dialysemittels elastisch verformbar ist.

15. Dialysesonde nach einem der Ansprüche 1 bis 14, **dadurch gekennzeichnet**, daß die Dialysemembran aus einem Material aus der Gruppe Cellophan, Cuprophan, Polykarbonat, Polyurethan oder Polyurethan mit eingebautem Polyäther besteht.

## Claims

1. A dialytic probe (1) with an ingoing and an outgoing line (9, 10) for the dialytic medium, comprising an insertion needle (2) for introducing the probe, which needle (2) is surrounded by a flexible plastic cannula (4), which in turn is surrounded, at least partly, by a tubular dialysing membrane (5) connected to the plastic cannula (4) at the distal end so as to form a fluid-tight seal, **characterized in that** a handle (8) is provided, which has separate ingoing and outgoing lines (9, 10) for feeding and draining the dialytic medium, and that at least two lumens (11, 12) are provided in the probe, which are connected to the ingoing and outgoing lines (9, 10), one of which lumens at least is bounded by the dialysing membrane (5), and that the lumen (11) of the plastic cannula (4) opening up after the insertion needle (2) has been removed, is connected to one of the ingoing and outgoing lines (9), and the lumen (12) between plastic cannula (4) and dialysing membrane (5) is connected to the other one of the ingoing and outgoing lines (10), and that the distal end (6) of the plastic cannula (4) has a lateral wall opening (13) leading into the lumen (12) between plastic cannula (4) and dialysing membrane (5).

2. A dialytic probe (1) with an ingoing and an outgoing line (9, 10) for the dialytic medium, comprising an insertion needle (2) for introducing the probe, which needle (2) is surrounded by a flexible plastic cannula (4), which in turn is surrounded, at least partly, by a tubular dialysing membrane (5) connected to the plastic cannula (4) at the distal end so as to form a fluid-tight seal, **characterized in that** a handle (8) is provided, which has separate ingoing and outgoing lines (9, 10) for feeding and draining the dialytic medium, and that at least two lumens (11, 12) are provided in the probe, which are connected to the ingoing and outgoing lines (9, 10), one of which lumens at least is bounded by the dialysing membrane (5), and that the lumen between plastic cannula (4) and dialysing membrane (5) is divided along the length of the dialytic probe (1), forming two lumens (15, 16) of essentially crescent-shaped cross-section, which two lumens (15, 16) have a flow-connection (17) at the distal end (6) of the plastic cannula (4) and a connection to one of the separate ingoing and outgoing lines (9, 10) each, and further that the lumen (11) of the plastic cannula (4) opening up after removal of the insertion needle (2) can be connected to a drug feeder line (18) in the handle (8).

3. A dialytic probe according to claim 2, **characterized in that** the dialysing membrane (5) is bonded to the plastic cannula (4) by glueing or welding, such that two beads or welds (19) run essentially along the length of the dialytic probe (1), and an annular bead or weld (20) is placed at the distal end (6), the longitudinal beads or welds (19) being separated from the annular bead or weld (20) by at least one gap establishing the flow connection (17) between the two lumens (15, 16).

4. A dialytic probe according to claim 2, **characterized in that** the dialysing membrane (5) is attached to two webs (21) running essentially along the length of the dialytic probe (1), and, possibly, to a distal, annular web (22) of the plastic cannula (4), the longitudinal webs (21) being separated from the annular web (22) by at least one gap establishing the flow connection (17) between the two lumens (15, 16).

5. A dialytic probe according to claim 4, **characterized in that** the dialysing membrane (5) is mechanically fastened to the longitudinal webs (21) and, possibly, the annular web (22), i.e., preferably by clamping.

6. A dialytic probe (1) with an ingoing and an outgoing line (9, 10) for the dialytic medium, comprising an insertion needle (2) for introducing the probe, which needle (2) is surrounded by a flexible plastic cannula (4), which in turn is surrounded, at least partly, by a tubular dialysing membrane (5) connected to the plastic cannula (4) at the distal end so as to form a fluid-tight seal, **characterized in that** a handle (8) is provided, which has separate ingoing and outgoing lines (9, 10) for feeding and draining the dialytic medium, and that at least two lumens (11, 12) are provided in the probe, which are connected to the ingoing and outgoing lines (9, 10), one of which lumens at least is bounded by the dialysing membrane (5), and that the plastic cannula (4) has an interior lumen (11) which opens up after the insertion needle (2) has been removed and which is connected to a drug feeder line (18), and that a further lumen (23) is provided in the wall of the plastic cannula (4), which is connected to one of the ingoing and outgoing lines (9), and that the lumen (12') between plastic cannula (4) and dialysing membrane (5) is connected to the other one of the ingoing and outgoing lines (10), the lumen (23) in the wall of the plastic cannula (4) having at least one wall opening (24) leading into the lumen (12') bounded by the dialysing membrane (5).

7. A dialytic probe (1) with an ingoing and an outgoing line (9, 10) for the dialytic medium, comprising an insertion needle (2) for introducing the probe, which needle (2) is surrounded by a flexible plastic cannula (4), which in turn is surrounded, at least partly, by a tubular dialysing membrane (5) connected to the plastic cannula (4) at the distal end so as to form a fluid-tight seal, **characterized in that** a handle (8) is provided, which has separate ingoing and outgoing lines (9, 10) for feeding and draining the dialytic medium, and that at least two lumens (28, 12') are provided in the probe, which are connected to the ingoing and outgoing lines (9, 10), one of which lumens at least is bounded by the dialysing membrane (5), and that the lumen (12') between plastic cannula (4) and dialysing membrane (5) is connected to one of the ingoing and outgoing lines (10), and that the insertion needle (2) is configured as a hollow needle up to a lateral wall opening (13' ) at the distal end, the other one of the ingoing and outgoing lines (9) being integrated into the needle handle (8), and the insertion needle (2) being retractable after the introduction of the probe, up to the point where its lateral wall opening (13') coincides with a distal wall opening (13) in the plastic cannula (4).

8. A dialytic probe according to claim 7, **characterized in that** the plastic cannula (4) is provided with another lateral wall opening (13'') in a distal region not covered by the membrane, which opening will coincide with the wall opening (13') of the insertion needle (2) after the latter has been moved axially.

9. A dialytic probe according to claim 7 or 8, **characterized in that** means (30, 31) are provided for the axial positioning of the insertion needle (2) as well as elements (32) for securing the insertion needle against turning.

10. A dialytic probe according to any of claims 1 to 9, **characterized in that** the handle (8) of the dialytic probe (1) contains the plastic cannula (4) in a centre bore (26), the handle (8) being tapered towards the distal end (6) of the plastic cannula (4), such that the dialysing membrane (5) may be slipped over the exterior face of the handle (8) and attached thereto.

11. A dialytic probe according to claim 10, **characterized in that** channels (27) are provided in the centre bore (26) of the handle (8), which channels (27), together with the inserted plastic cannula (4), form flow connections between at least one of the ingoing and outgoing lines (9, 10) and at least one lumen (12; 12'; 15, 16) between plastic cannula (4) and dialysing membrane (5).

12. A dialytic probe according to claim 10 or 11, **characterized in that** the end of the dialysing membrane (5) facing the handle (8) has a larger diameter than at the distal end (6) of the plastic cannula (4).

13. A dialytic probe according to any of claims 1 to 12, **characterized in that** the handle (8) of the probe (1) is furnished with a flexible stopper (14) pierced by the insertion needle (2), which stopper (14) plugs the probe (1) automatically in the area of the handle (8) after removal of the insertion needle (2).

14. A dialytic probe according to any of claims 1 to 13, **characterized in that** the dialysing membrane (5) is made of resilient material permitting elastic deformation if subject to the pressure of the dialytic medium.

15. A dialytic probe according to any of claims 1 to 14, **characterized in that** the dialysing membrane (5) is made of a material from the group of cellophane, cuprophane, polycarbonate, polyurethane, or polyurethane with incorporated polyether.

## Revendications

1. Sonde de dialyse (1) avec une conduite d'arrivée et de départ (9, 10) pour le produit de dialyse, qui comporte une aiguille d'introduction (2) destinée à introduire la sonde, qui est entourée par une canule en matière plastique (4) flexible, laquelle de son côté est entourée au moins en partie par une membrane de dialyse (5) en forme de tuyau, la membrane de dialyse (5) étant reliée à l'extrémité distale, de manière étanche aux liquides, avec la canule en matière plastique (4), caractérisée en ce qu'il est prévu une poignée (8) qui comporte des conduites d'arrivée et de départ (9, 10) séparées pour le produit de dialyse, et en ce que dans la sonde sont guidées au moins deux cavités (11, 12) reliées aux conduites d'arrivée et de départ (9, 10), une cavité au moins étant délimitée par la membrane de dialyse (5), en ce que la cavité (11), libérée après enlèvement de l'aiguille d'introduction (2), de la canule en matière plastique (4) est reliée avec l'une des conduites d'arrivée et de départ (9) et la cavité (12) entre la canule en matière plastique (4) et la membrane de dialyse (5) communique avec l'autre conduite d'arrivée et de départ (10), et en ce que l'extrémité distale (6) de la canule en matière plastique (4) présente une ouverture de paroi (13) latérale vers la cavité (12) entre la canule en matière plastique (4) et la membrane de dialyse (5).

2. Sonde de dialyse (1) avec une conduite d'arrivée et de départ (9, 10) pour le produit de dialyse, qui comporte une aiguille d'introduction (2) destinée à introduire la sonde, qui est entourée par une canule en matière plastique (4) flexible, laquelle de son côté est entourée au moins en partie par une membrane de dialyse (5) en forme de tuyau, la membrane de dialyse (5) étant reliée à l'extrémité distale, de manière étanche aux liquides, avec la canule en matière plastique (4), caractérisée en ce qu'il est prévu une poignée (8) qui comporte des conduites d'arrivée et de départ (9, 10) séparées pour le produit de dialyse, et en ce que dans la sonde sont guidées au moins deux cavités (15, 16) reliées aux conduites d'arrivée et de départ (9, 10), une cavité au moins étant délimitée par la membrane de dialyse (5), en ce que la cavité comprise entre la canule en matière plastique (4) et la membrane de dialyse (5) est partagée sensiblement dans la direction longitudinale de la sonde de dialyse (1), de sorte qu'il se forme deux cavités (15, 16) de section transversale sensiblement en forme de demi-lune, les deux cavités (15, 16) présentent dans la zone de l'extrémité distale (6) de la canule en matière plastique (4), une liaison d'écoulement (17) et communiquent chacune avec l'une des conduites (9, 10) séparées d'arrivée et de départ, et en ce que la cavité (11), libérée après enlèvement de l'aiguille d'introduction (2), de la canule en matière plastique, peut être reliée avec une arrivée de médicament (18) dans la poignée (8).

3. Sonde de dialyse selon la revendication 2, caractérisée en ce que la membrane de dialyse (5) est assemblée à la canule en matière plastique (4) par deux points de collage ou de soudage (19), s'étendant sensiblement dans la direction longitudinale de la sonde de dialyse (1) et par un point de collage ou de soudage (20) annulaire, distal, entre les points de collage ou de soudage (19), s'étendant dans la direction longitudinale, et le point de collage ou de soudage (20) annulaire étant formé au moins un interstice par lequel est réalisée la liaison d'écoulement (17) entre les deux cavités (15, 16).

4. Sonde de dialyse selon la revendication 2, caractérisée en ce que la membrane de dialyse (5) est fixée sur deux cloisons (21) s'étendant sensiblement dans la direction longitudinale de la sonde de dialyse (1) et sur éventuellement une cloison (22) annulaire distale de la canule en matière plastique (4), entre les cloisons (21) s'étendant dans la direction longitudinale et la cloison (22) annulaire étant formé au moins un interstice par lequel est réalisée la liaison d'écoulement (17) entre les deux cavités (15, 16).

5. Sonde de dialyse selon la revendication 4, caractérisée en ce que la membrane de dialyse (5) est fixée mécaniquement, de préférence par serrage, sur les cloisons (21) s'étendant dans la direction longitudinale et éventuellement sur la cloison (22) annulaire.

6. Sonde de dialyse (1) comportant une conduite d'arrivée et de départ (9, 10) pour le produit de dialyse, qui comporte une aiguille d'introduction (2) destinée à introduire la sonde, qui est entourée par une canule en matière plastique (4), laquelle de son côté est entourée au moins en partie par une membrane de dialyse (5) en forme de tuyau, la membrane de dialyse (5) étant reliée à l'extrémité distale, de manière étanche aux liquides, avec la canule en matière plastique (4), caractérisée en ce qu'il est prévu une poignée (8) qui comporte des conduites d'arrivée et de départ (9, 10) séparées pour le produit de dialyse, et en ce que dans la sonde sont guidées au moins deux cavités (23, 12') reliées aux conduites d'arrivée et de départ (9, 10), une cavité au moins étant délimitée par la membrane de dialyse (5), en ce que la canule en matière plastique (4) présente une cavité (11) intérieure, libérée après enlèvement de l'aiguille d'introduction (2), qui est reliée avec une conduite d'arrivée de médicament (18), en ce que dans la paroi de la canule en matière plastique (4) il est prévu une autre cavité (23) qui communique avec l'une des conduites d'arrivée et de départ (9), et en ce que la cavité (12') entre la canule en matière plastique (4) et la membrane de dialyse (5) est reliée avec l'autre conduite d'arrivée et de départ (10), la cavité (23) de la paroi de la canule en matière plastique (4) présentant au moins une ouverture de paroi (24) vers la cavité (12') limitée par la membrane de dialyse (5).

7. Sonde de dialyse (1) comportant une conduite d'arrivée et de départ (9, 10) pour le produit de dialyse, qui comporte une aiguille d'introduction (2) destinée à introduire la sonde, qui est entourée par une canule en matière plastique (4), laquelle de son côté est entourée au moins en partie par une membrane de dialyse (5) en forme de tuyau, la membrane de dialyse (5) étant reliée à l'extrémité distale, de manière étanche aux liquides, avec la canule en matière plastique (4), caractérisée en ce qu'il est prévu une poignée (8) qui comporte des conduites d'arrivée et de départ (9, 10) séparées pour le produit de dialyse, et en ce que dans la sonde sont guidées au moins deux cavités (28, 12') reliées aux conduites d'arrivée et de départ (9, 10), une cavité au moins étant délimitée par la membrane de dialyse (5), en ce que la cavité (12') entre la canule en matière plastique (4) et la membrane de dialyse (5) communique avec l'une des conduites d'arrivée et de départ (10), en ce que l'aiguille d'introduction (2), jusqu'à une ouverture de paroi (13') latérale distale, est réalisée sous la forme d'une aiguille creuse qui, à l'extrémité de saisie, comporte l'autre des conduites d'arrivée et de départ (9), l'aiguille d'introduction (2) pouvant être retirée après l'introduction de la sonde, jusqu'à ce que son ouverture de paroi (13') latérale coïncide avec une ouverture de paroi (13) distale de la canule en matière plastique (4).

8. Sonde de dialyse selon la revendication 7, caractérisée en ce que la canule en matière plastique (4) présente, dans une zone distale, sans membrane, une autre ouverture de paroi (13') latérale qui, après un déplacement axial de l'aiguille d'introduction (2), coïncide avec son ouverture de paroi (13').

9. Sonde de dialyse selon la revendication 7 ou 8, caractérisée en ce qu'il est prévu des moyens (30, 31) pour le positionnement axial de l'aiguille d'introduction (2) ainsi que des éléments (32) pour empêcher la rotation de l'aiguille d'introduction.

10. Sonde de dialyse selon l'une des revendications 1 à 9, caractérisée en ce que la poignée (8) de la sonde de dialyse (1) loge la canule en matière plastique (4) dans un alésage (26) central, la poignée (8) se rétrécissant en direction de l'extrémité distale (6) de la canule en matière plastique (4) ce qui fait que la membrane de dialyse (5) peut coulisser sur la surface extérieure de la poignée (8) et y être fixée.

11. Sonde de dialyse selon la revendication 10, caractérisée en ce que l'alésage (26) central de la poignée (8) présente des découpes (27) qui forment avec la canule en matière plastique (4) insérée des liaisons d'écoulement entre au moins l'une des conduites d'arrivée et de départ (9, 10) et au moins une cavité (12 ; 12' ; 15, 16) entre la canule en matière plastique (4) et la membrane de dialyse (5).

12. Sonde de dialyse selon la revendication 10 ou 11, caractérisée en ce que la membrane de dialyse (5) présente à l'extrémité tournée vers la poignée (8), un plus grand diamètre qu'à l'extrémité distale (6) de la canule en matière plastique (4).

13. Sonde de dialyse selon l'une des revendications 1 à 12, caractérisée en ce que la poignée (8) de la sonde de dialyse (1) loge un bouchon (14) élastique, traversé par l'aiguille d'introduction (2), lequel bouchon ferme automatiquement la sonde de dialyse (1), après enlèvement de l'aiguille d'introduction (2), dans la zone de la poignée (8).

14. Sonde de dialyse selon l'une des revendications 1 à 13, caractérisée en ce que la membrane de dialyse (5) est réalisée dans un matériau extensible et peut être déformée élastiquement par la pression du produit de dialyse.

15. Sonde de dialyse selon l'une des revendications 1 à 14, caractérisée en ce que la membrane de dialyse est réalisée dans un matériau du groupe cellophane, cuprophane, polycarbonate, polyuréthanne ou polyuréthanne avec polyéther incorporé.
